# EUROPEAN PATENT APPLICATION

(11) **EP 4 781 927 A1**
(43) Date of publication of application: **29.07.2026**
(21) Application number: 24867125.7
(22) Date of filing: 02.08.2024
(51) Int. Cl.: A61B 17/29

(54) **PUSHING DEVICE FOR MAGNETICALLY CONTROLLED FORCEPS AND MAGNETICALLY CONTROLLED FORCEPS**

(30) Priority: 22.09.2023 CN 202311238159
(71) Applicant: Shaanxi Micropre Medical Biotechnology Co., Ltd., Xi'an, Shaanxi 710000 (CN)
(72) Inventor: LI, Hongling, Shaanxi 710000 (CN); JIA, Jintong, Shaanxi 710000 (CN); SONG, Tao, Shaanxi 710000 (CN); HE, Zhenan, Shaanxi 710000 (CN)
(74) Representative: RGTH
(86) International application number: PCT/CN2024/109443
(87) International publication number: WO 2025/060708

(57) **Abstract**

The present invention provides a pushing device for a magnetically controlled forceps and the magnetically controlled forceps. The pushing device for the magnetically controlled forceps includes an actuation module, a sliding member, and a first magnet. The sliding member is located around a periphery of the first magnet, and the actuation module is in driving connecting with the sliding member to drive the sliding member to move back and forth. A movable jaw of a jaw portion is driven by the sliding member disposed around the periphery of the first magnet and abutting against the movable jaw, and a position where the sliding member abuts against the movable jaw is offset from a position of a hinge axis of a fixed jaw and the movable jaw, so that the movable jaw is driven more precisely with less labor. In addition, there is no need to open a hole in the middle of the jaw portion, which can ensure the firmness of magnetic attraction and cooperation between the jaw portion and the shaft portion, eliminates the need for an additional fixing structure, and greatly reduces an error rate.

## Description

### TECHNICAL FIELD

The present invention relates to the field of surgical instruments, and in particular to a pushing device for a magnetically controlled forceps and the magnetically controlled forceps.

### BACKGROUND

With the rapid development of laparoscopic and Da Vinci robotic surgery, minimally invasive techniques and aesthetic surgery have become key objectives in modern surgical practice. However, it is undeniable that any technological innovation requires the creation of an adequate surgical workspace to implement optimal exposure of a surgical site. A crucial issue is how to increase a traction force at the surgical site without damaging an abdominal wall, to complete a surgery successfully.

Main challenges of previous classic single-incision laparoscopic surgery (SILS) and natural orifice transluminal endoscopic surgery (NOTES) are ergonomic limitations, the loss of triangulation mechanics, and the chopstick effect, which result in impaired operational triangulation and poor exposure of the surgical site. In contrast, existing magnetic anchoring and guidance system technologies provide adjustable traction by using an external handheld magnet to manipulate intra-abdominal components for surgical workspace traction. This strategy allows the use of a single entry point (through an abdominal wall or via a natural orifice) or a controlled number of entry points as operative paths for multiple instruments, thereby reducing abdominal wall trocar use during laparoscopic surgery.

CN 105392416 B discloses a grasper with magnetic positioning. The grasper (namely, a jaw portion) and a delivery device (namely, a shaft portion) are detachably assembled via magnetic attraction. After the grasper and the delivery device are magnetically fixed, a brake rod 114 of the delivery device passes through an inner cavity 212 of a cylindrical portion 210 of the grasper 200 to actuate the grasper 200. Specifically, the brake rod 114 passes through the inner cavity 212 of the cylindrical portion 210 and abuts against a proximal arm 220 of a first jaw 202, thereby driving the first jaw 202 to operate. This solution has the following drawbacks: 1. a hinge axis 208 of the first jaw 202 and the second jaw 204 is essentially on an extended line of the brake rod 114, namely, the brake rod 114 and the hinge axis 208 are almost collinear, resulting in a minimal force arm, and the brake rod 114 abuts against the proximal arm 220 and drives the first jaw 202 to rotate, requiring a surgeon to exert an extremely great force, increasing a surgical risk, a large fixation force is required to ensure that the grasper and the delivery device are not separated before the first jaw 202 is fully opened, and a locking sheath 116 is used for auxiliary fixation in this solution; 2. the grasper 200 requires an additional space (namely, the inner cavity 212 of the cylindrical portion 210) for the brake rod 114 to pass through. In this way, an area for magnetic attraction and fixation with the delivery device is greatly reduced, resulting in weak magnetic attraction. In this solution, the grasper 200 is additionally locked by the locking sheath 116, whereby a complex and precision-fitted structure is disposed within an extremely limited space. Consequently, a manufacturing difficulty is increased, and a malfunction is more likely to occur, which is unacceptable in surgery.

### INVENTION DISCLOSURE

To address the above issues, the present invention provides a pushing device for a magnetically controlled forceps and the magnetically controlled forceps.

To achieve the above objective, a technical solution provided by the present invention is as follows:
A pushing device for a magnetically controlled forceps, including an actuation module, a sliding member, and a first magnet, where the sliding member is located around a periphery of the first magnet, and the actuation module is in driving connection with the sliding member to drive the sliding member to move back and forth.

Further, the actuation module includes a central rod assembly and a pull rod assembly; the first magnet is assembled at a front end of the pull rod assembly to move back and forth when driven by the pull rod assembly; and the sliding member is located around the periphery of the first magnet and connected to the central rod assembly to move back and forth when driven by the central rod assembly.

Further, the pushing device for the magnetically controlled forceps further includes a support tube, and the central rod assembly, the pull rod assembly, and the first magnet are all disposed in the support tube.

Further, the sliding member is a sliding tube, and the sliding tube is slidably sleeved on an outer wall of the support tube.

Further, the central rod assembly and the pull rod assembly are arranged in a push-pull linkage; and the pull rod assembly has a forward driving force, and the central rod assembly has a rearward driving force.

Further, the pull rod assembly includes a pull rod and a first compression spring, the pull rod is disposed in the first magnet and abuts against a front end of the first magnet, and the first compression spring applies a forward elastic force to the first magnet.

Further, the central rod assembly includes a central rod, a central rod limit buckle, and a second compression spring, the central rod is fixedly connected to the central rod limit buckle, the central rod is connected to the sliding member, and the second compression spring applies a rearward elastic force to the central rod limit buckle and the central rod.

Further, a limit notch extending in a front-rear direction is formed on the central rod assembly, and the pull rod assembly is provided with a pin and is disposed in the limit notch through the pin, thereby implementing a push-pull linkage between the central rod assembly and the pull rod assembly.

Further, the pushing device for the magnetically controlled forceps further includes a movable handle, and the movable handle acts on the actuation module to actuate the actuation module.

A magnetically controlled forceps, including a jaw portion and a shaft portion that are separable, where the jaw portion includes a fixed jaw, a movable jaw, and a second magnet, the movable jaw is hinged to the fixed jaw and is provided with a driving arm; the second magnet is connected to the fixed jaw; the shaft portion is the pushing device for the magnetically controlled forceps; the first magnet is configured to magnetically engage and fix with the second magnet, to fix the jaw portion and the shaft portion; and the actuation module drives a sliding member to pass over a periphery of the second magnet and abut against the driving arm of the movable jaw to actuate the movable jaw.

Further, the second magnet is a solid permanent magnet, and the second magnet is embedded in a rear end of the fixed jaw; and the first magnet is a permanent magnet, and the first magnet and the second magnet are engaged and fixed by opposite poles.

The technical solutions of the present invention have the following significant beneficial effects.
1. The movable jaw of the jaw portion is driven by the sliding member disposed around the periphery of first magnet and abutting against the movable jaw, and a position where the sliding member abuts the movable jaw is offset from a position of a hinge axis of a fixed jaw and the movable jaw, with a power arm excessively greater than a power arm in the existing solution, so that the movable jaw is driven more precisely with less labor. In addition, there is no need to open a hole in the middle of the jaw portion, which ensures the firmness of magnetic coupling between the jaw portion and the shaft portion, eliminates the need for an additional fixing structure, and provides a simple structure that is easy to process, greatly reducing an error rate, and improving surgical safety.
2. The central rod assembly and the pull rod assembly are arranged in a push-pull linkage; and the pull rod assembly has a forward driving force, and the central rod assembly has a rearward driving force. With the interaction of the forward driving force of the pull rod assembly, the rearward driving force of the central rod assembly, and the magnetic attraction of the first magnet, balance and a stable state can be maintained. When an external force is applied, the original balance is disrupted and quickly switches to another state; this enables more effortless actuation. With the cooperation of the movable handle, single-handed rapid switching of actions such as magnetic engagement of the jaw portion and the pushing device, tissue clamping by the jaw portion, and separation of the jaw portion and pushing device can be implemented, providing a simpler, more precise, and efficient operation.

### BRIEF DESCRIPTION OF DRAWINGS

FIG. 1 is a schematic view of an external appearance of a pushing device for a magnetically controlled forceps according to an embodiment;
FIG. 2 is an exploded schematic structural view of the pushing device for the magnetically controlled forceps according to an embodiment;
FIG. 3 is a schematic structural view of a jaw portion and a shaft portion of the magnetically controlled forceps in a separated state according to an embodiment;
FIG. 4 is a schematic structural view of the jaw portion and the shaft portion of the magnetically controlled forceps in an assembled state according to an embodiment;
FIG. 5 is a sectional view of the jaw portion and the shaft portion of the magnetically controlled forceps in the assembled state according to an embodiment;
FIG. 6 is a schematic enlarged view of an area A in FIG. 5; and
FIG. 7 is a partial sectional view of the jaw portion, of the magnetically controlled forceps, driven to be in an opened state according to an embodiment.

### DESCRIPTION OF EMBODIMENTS

To further illustrate each embodiment, the present invention is provided with accompanying drawings. These drawings are a part of the disclosure of the present invention, and are mainly used to illustrate the embodiments and can be used with reference to related descriptions in the specification to explain operating principles of the embodiments. With reference to the content, those skilled in the art shall understand other possible implementations and advantages of the present invention. Components in the drawings are not drawn to scale, and similar component symbols are usually used to represent similar components.

The present invention is further illustrated below in conjunction with accompanying drawings and specific embodiments.

### Embodiment 1

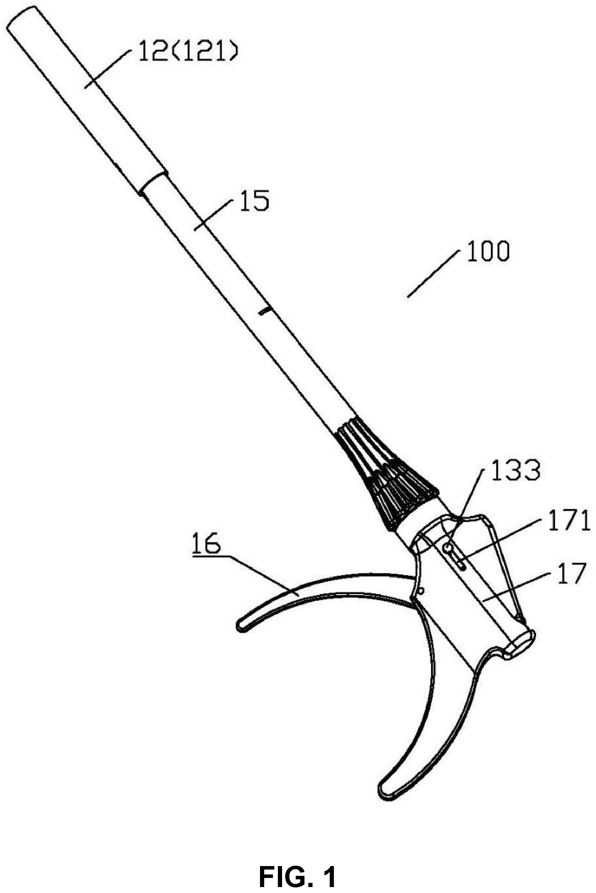

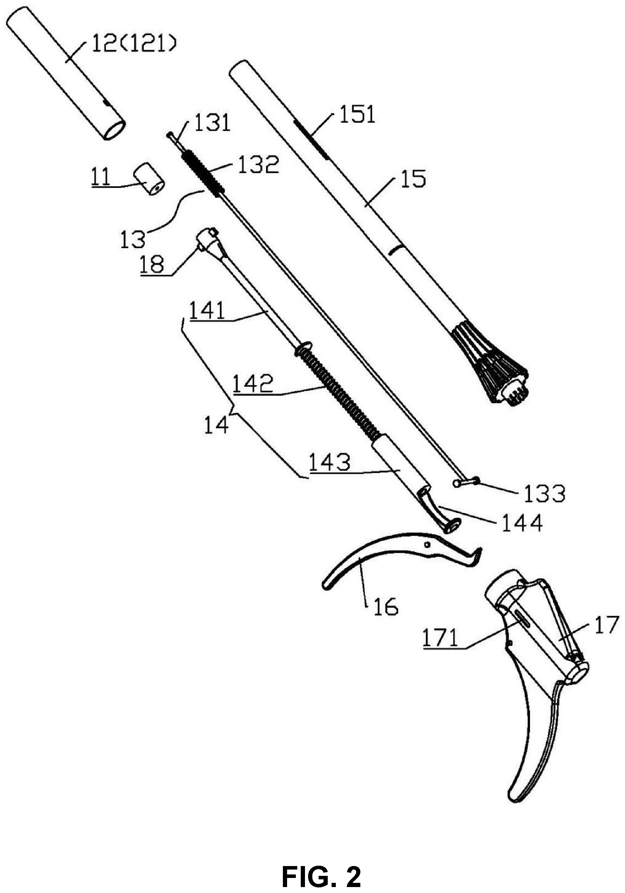

FIG. 1 and FIG. 2 show a pushing device for a magnetically controlled forceps provided in this embodiment, namely, a shaft portion 100 of the magnetically controlled forceps, including an actuation module, a sliding member 12, and a first magnet 11. The actuation module includes a central rod assembly 14 and a pull rod assembly 13; the first magnet 11 is assembled at a front end of the pull rod assembly 13, namely, the first magnet 11 is driven back and forth by the pull rod assembly 13; and the sliding member 12 is located around a periphery of the first magnet 11 and is connected to the central rod assembly 14 to move back and forth when driven by the central rod assembly 14.

Specifically, the sliding member 12 is located around the periphery of the first magnet 11, meaning that on a projection plane perpendicular to a front-rear direction (namely, equivalent to a radial plane of a support tube 15 described below), the sliding member 12 is located around the periphery of the first magnet 11.

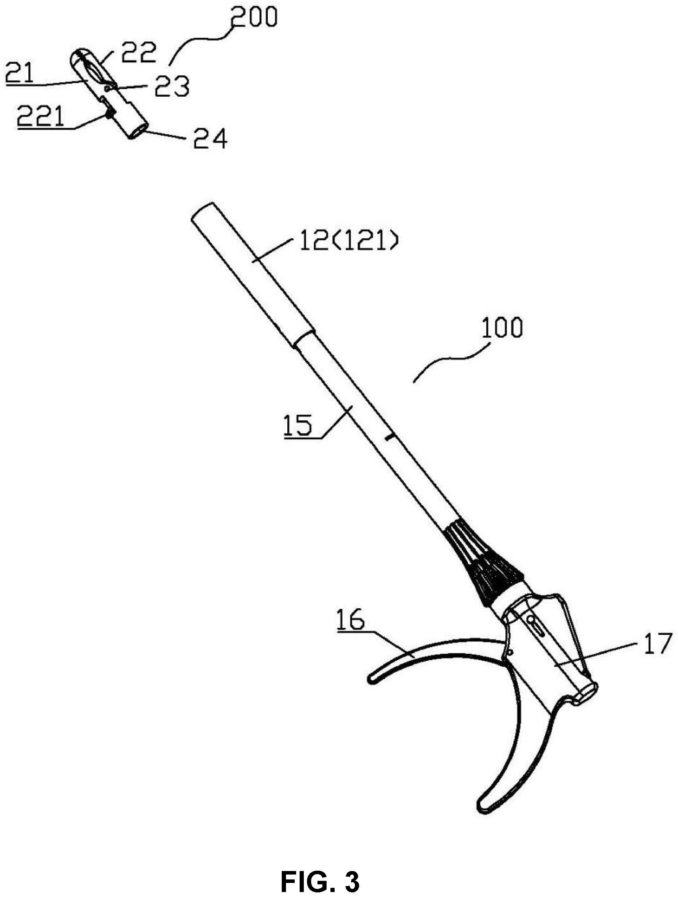

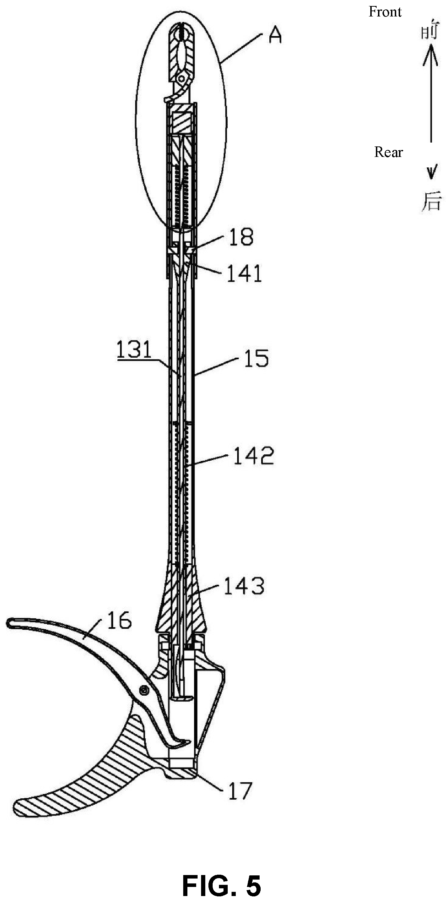

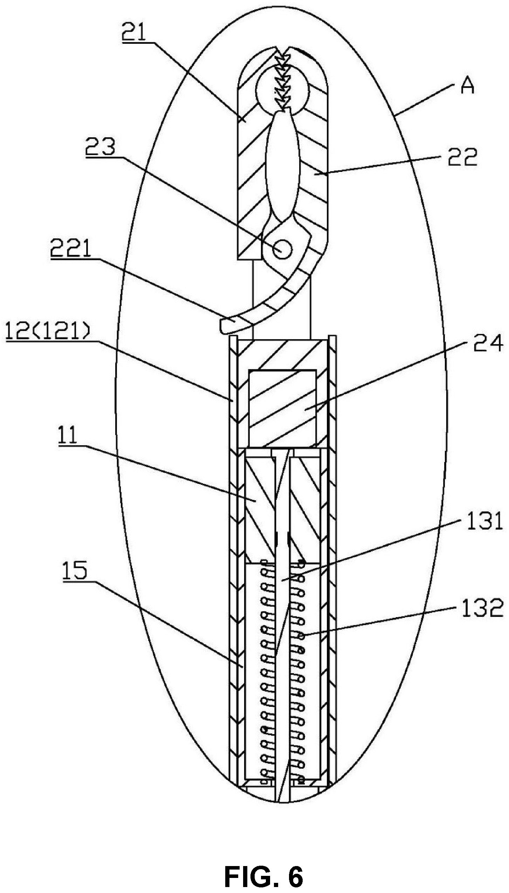

Still refer to FIG. 3 to FIG. 7. This embodiment also provides a magnetically controlled forceps, including a jaw portion 200 and the shaft portion 100 that can be separated. The jaw portion 200 includes a fixed jaw 21, a movable jaw 22, and a second magnet 24. The movable jaw 22 is hinged to the fixed jaw 21 and is provided with a driving arm 221. Specifically, the movable jaw 22 is hinged to the fixed jaw 21 through a hinge axis 23, and the driving arm 221 of the movable jaw 22 is of an arc-shaped structure. In a closed state shown in FIG. 5 and FIG. 6, the driving arm 221 bends around a position of the hinge axis 23 from one side and extends to the other side. The second magnet 24 is connected to the fixed jaw 21.

The shaft portion 100 is the pushing device for the magnetically controlled forceps as described above; forward or backward movement of the pull rod assembly 13 causes the first magnet 11 to be magnetically engaged with or detached from the second magnet 24 of the jaw portion 200. To be specific, when the pull rod assembly 13 moves forward, the first magnet 11 is driven to move forward to magnetically engage the second magnet 24 of the jaw portion 200, thereby fixing the jaw portion 200 and the shaft portion 100 via magnetic attraction between the first magnet 11 and the second magnet 24. When the pull rod assembly 13 moves backward, the first magnet 11 is driven to move backward, causing the first magnet 11 to move away from the second magnet 24 and thus detach, implementing separation of the jaw portion 200 and the shaft portion 100.

The forward movement of the central rod assembly 14 drives the sliding member 12 to abut against the driving arm 221 of the movable jaw 22 around a periphery of the second magnet 24, thereby actuating the movable jaw 22 to move. To be specific, after the jaw portion 200 and the shaft portion 100 are fixed by magnetic attraction between the first magnet 11 and the second magnet 24, the central rod assembly 14 further drives the sliding member 12 to abut against the driving arm 221 of the movable jaw 22 around the periphery of the second magnet 24, thereby actuating the movable jaw 22 to move. In this specific embodiment, the fixed jaw 21 and the movable jaw 22 are opened.

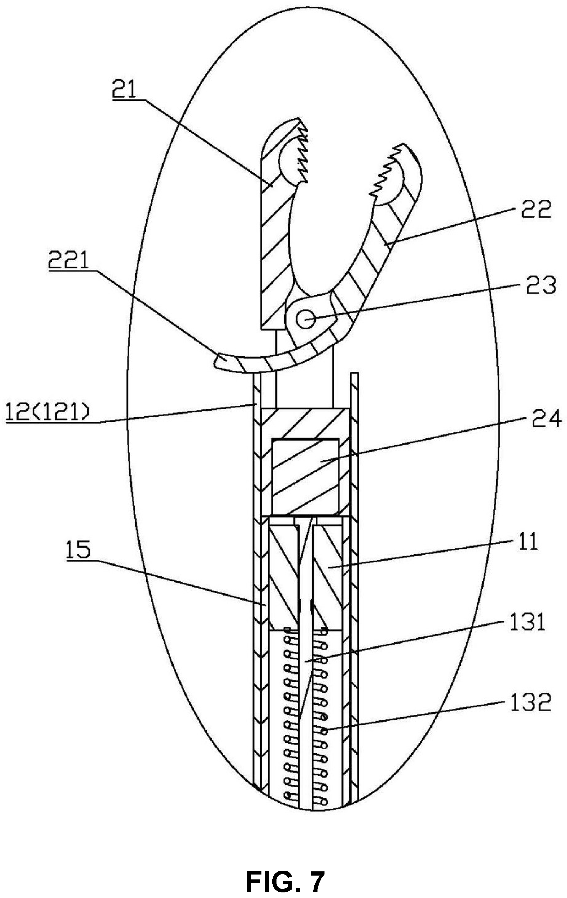

In the above solution, the movable jaw 22 of the jaw portion 200 is driven by the sliding member 12 disposed around the periphery of the first magnet 11 and abutting against the movable jaw, and a position where the sliding member 12 abuts against the movable jaw 22 is offset from a position of the hinge axis 23 of the fixed jaw 21 and the movable jaw 22. As shown in FIG. 6 and FIG. 7, the hinge axis 23 of the fixed jaw 21 and the movable jaw 22 is aligned with a center of the first magnet 11, while the sliding member 12 is disposed around the periphery of the first magnet 11. Therefore, the position where the sliding member 12 abuts against the driving arm 221 is offset from a position of the hinge axis 23, resulting in a force arm much greater than a force arm in an existing solution. In this way, the actuation of the movable jaw 22 is more precise and effortless, and a movement response of the movable jaw 22 is more immediate.

In addition, there is no need to open a hole in the middle of the jaw portion 200 (namely, the second magnet 24 can be of a solid structure), thus ensuring the reliability of magnetic coupling between the jaw portion 200 and the shaft portion 100, without the need for an additional fixation structure. The structure is simple and easy to manufacture, greatly reducing an error rate and improving surgical safety.

Specifically, the pushing device (shaft portion 100) for the magnetically controlled forceps further includes a support tube 15, the central rod assembly 14, the pull rod assembly 13, and the first magnet 11 are all disposed in the support tube 15, which better facilitates assembly and serves to protect the actuation module, preventing the central rod assembly 14, the pull rod assembly 13, and the first magnet 11 from being exposed and easily damaged by accidental contact or affected by an external structure.

In a preferred solution, in this embodiment, the sliding member 12 is a sliding tube 121, and the sliding tube 121 is slidably sleeved on an outer wall of the support tube 15. A tubular structure of the sliding tube 121 provides excellent guidance when fitted with the support tube 15, preventing deviation, and ensuring fuller contact with the driving arm 221 of the movable jaw 22. Of course, in other embodiments, the sliding member 12 may alternatively be an arc-shaped sheet structure, or the like.

An elongated clearance slot 151 extending in a front-rear direction is formed on the support tube 15, and a pin member 18 is disposed in the elongated clearance slot 151 to connect the central rod assembly 14 and the sliding member 12, thereby implementing a connection between the central rod assembly 14 and the sliding member 12.

Specifically, the second magnet 24 is a solid permanent magnet, and the second magnet 24 is embedded in a rear end of the fixed jaw 21; and the first magnet 11 is a permanent magnet, and the first magnet 11 and the second magnet 24 are magnetically engaged and fixed by opposite poles. Magnetic fixation is implemented by two permanent magnets, providing a more secure magnetic attraction, and offering an excellent self-aligning capability, and ensuring connection accuracy between the jaw portion 200 and the shaft portion 100. Of course, in other embodiments, one of the first magnet 11 and the second magnet 24 may be a permanent magnet, and the other may be a magnetically permeable member, so that magnetic attraction and fixation between the permanent magnet and the magnetically permeable member can also be implemented. However, it may be necessary to add a positioning structure to ensure the connection accuracy between the jaw portion 200 and the shaft portion 100.

In one preferred solution, in this embodiment, the central rod assembly 14 and the pull rod assembly 13 are configured in a push-pull linkage; a driving force of the central rod assembly 14 is greater than a driving force of the pull rod assembly 13 and less than a sum of the driving force of the pull rod assembly 13 and a magnetic engagement force of the first magnet 11 when fully magnetically engaged; and the pull rod assembly 13 has a forward driving force, and the central rod assembly 14 has a rearward driving force. Balance can be maintained under the interaction of the forward driving force of the pull rod assembly 13, the rearward driving force of the central rod assembly 14, and a magnetic attraction force of the first magnet 11, keeping a stable state. When an external force is applied, an original balanced state is disrupted and quickly switches to another balanced state, making the driving force more effortless and operation more precise and efficient.

Specifically, the pull rod assembly 13 includes a pull rod 131 and a first compression spring 132. The pull rod 131 is disposed in the first magnet 11 and abuts against a front end of the first magnet 11. The first compression spring 132 applies a forward elastic force to the first magnet 11. In this way, the pull rod assembly 13 has a forward driving force. When the jaw portion 200 is assembled, an external force is applied as a forward thrust, and the forward driving force is greater than the rearward driving force. The first compression spring 132 pushes the first magnet 11 rapidly forward for magnetic attraction and fixation. The structure is simple and easy to implement.

The central rod assembly 14 includes a central rod 141, a central rod limit buckle 143, and a second compression spring 142. The central rod 141 is fixedly connected to the central rod limit buckle 143. The central rod 141 is connected to the sliding member 12. The second compression spring 142 applies a rearward elastic force to the central rod limit buckle 143 and the central rod 141, so that the central rod assembly 14 has a rearward driving force.

Specifically, the push-pull linkage between the central rod assembly 14 and the pull rod assembly 13 is implemented as follows: a limit notch 144 extending in a front-rear direction is formed on the central rod assembly 14 (specifically the central rod limit buckle 143), the pull rod assembly 13 (specifically the pull rod 131) is provided with a pin 133 and is disposed in the limit notch 144 through the pin 133, and a range of movement of the pin 133 within the limit notch 144 defines a separate movement range of the central rod assembly 14 or the pull rod assembly 13. When the pin 133 abuts against the limit notch 144, further movement by either side drives the other side to move synchronously. Therefore, the push-pull linkage between the central rod assembly 14 and the pull rod assembly 13 is implemented. The structure is simple and easy to implement.

The above specific structure of the central rod assembly 14 and the pull rod assembly 13 and the manner for push-pull linkage are one of preferred implementations of this solution. Of course, other embodiments are not limited thereto.

The pushing device for the magnetically controlled forceps further includes a movable handle 16. The movable handle 16 corresponds to the actuation module to drive the central rod assembly 14 and/or the pull rod assembly 13 to move forward. Specifically, in this embodiment, there is one movable handle 16, which corresponds to the central rod assembly 14. The movable handle 16 synchronously controls the movement of the pull rod assembly 13 by driving the central rod assembly 14, thereby simplifying the structure.

Further, the pushing device for the magnetically controlled forceps further includes a handheld portion 17. The handheld portion 17 is mounted at a rear end of the support tube 15. The movable handle 16 is hinged to the handheld portion 17. The handheld portion 17 is provided with a limit slot 171 extending in a front-rear direction. The pin 133 of the pull rod assembly 13 passes through the limit notch 144 and then passes through the limit slot 171, and is exposed on an outer surface of the handheld portion 17. This facilitates a pull-back operation of the pull rod assembly 13. To be specific, when it is necessary to disengage the jaw portion 200, the exposed pin 133 can be pulled backward, thereby driving the pull rod assembly 13 and the first magnet 11 to move rearward.

Further, the central rod 141 is a hollow rod structure, and the pull rod 131 is disposed in the central rod 141. The structure is compact and does not interfere with the second compression spring 142.

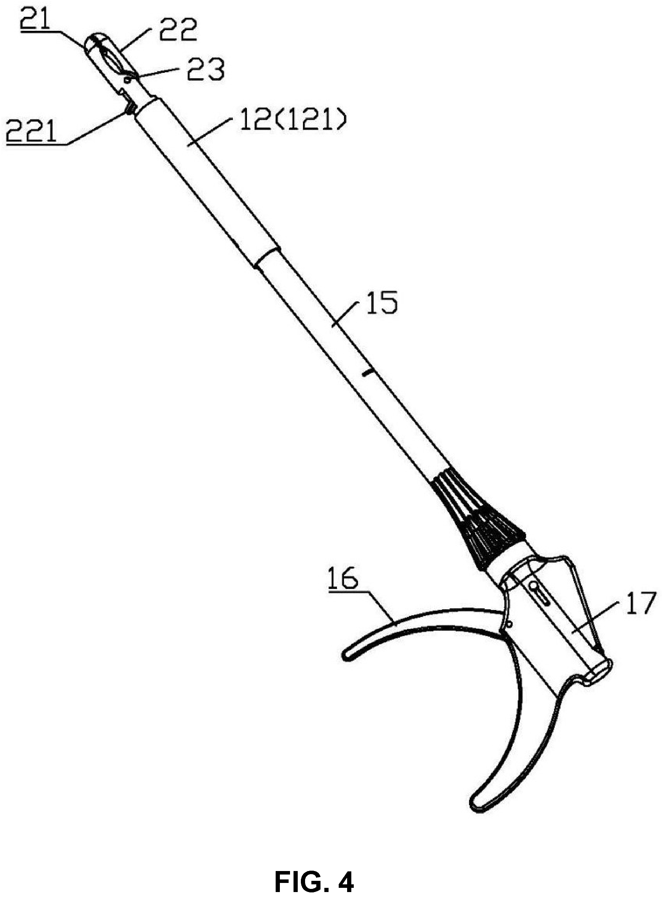

An operation process of the magnetically controlled forceps is as follows.
1. In an initial state, the jaw portion 200 and the shaft portion 100 are in a separated state. As shown in FIG. 3, the first magnet 11 is not magnetically engaged, so there is no magnetic engagement force. The rearward driving force of the central rod assembly 14 (an elastic force of the second compression spring 142) is greater than the forward driving force of the pull rod assembly 13 (the elastic force of the first compression spring 132). The central rod assembly 14, the pull rod assembly 13, and the first magnet 11 are held at rear positions.
2. When the jaw portion 200 needs to be assembled, the shaft portion 100 is aligned with the jaw portion 200. Because the first magnet 11 and the second magnet 24 have an initial magnetic attraction, when the shaft portion 100 approaches the jaw portion 200, the jaw portion 200 can quickly combine with the shaft portion 100. However, in this case, a magnetic engagement force is weak, and the jaw portion 200 and the shaft portion 100 are easily separated.
   In this case, an external force is applied to the movable handle 16 to push the central rod assembly 14 or the pull rod assembly 13. Under the action of the external force, the central rod assembly 14 overcomes a self-rearward driving force and moves forward. In this case, the pull rod assembly 13 drives the first magnet 11 to move forward when driven by a self-forward driving force. The first magnet 11 approaches and is then quickly magnetically engaged and fixed to the second magnet 24 of the jaw portion 200. The first magnet 11 and the second magnet 24 are completely magnetically engaged. In this case, a sum of the forward driving force of the pull rod assembly 13 and the magnetic engagement force between the first magnet 11 and the second magnet 24 is greater than the rearward driving force of the central rod assembly 14, and the jaw portion 200 and the shaft portion 100 remain in an assembled state, as shown in FIG. 4 to FIG. 6. The second magnet 24 has reached the foremost position. Continuing to drive the central rod assembly 14 forward does not affect the pull rod assembly 13. In this case, the movable handle 16 is pressed again, and the central rod assembly 14 and the sliding member 12 are continuously driven forward, so that the sliding member 12 abuts against the driving arm 221 of the movable jaw 22, causing the movable jaw 22 to open, as shown in FIG. 7. After the external force is removed, the central rod assembly 14 moves rearward under the self-rearward driving force until the central rod assembly 14 contacts the pull rod assembly 13 again.
3. When the jaw portion 200 and the shaft portion 100 need to be separated, a rearward external force is applied to the pull rod assembly 13, namely, the exposed pin 133 is pulled backward, causing the pull rod assembly 13 and the first magnet 11 to move rearward. The first magnet 11 is away from the second magnet 24, and the magnetic engagement force between the first magnet 11 and the second magnet 24 decreases. When the forward driving force (the sum of the driving force of the pull rod assembly and the magnetic engagement force of the first magnet) is less than the rearward driving force (the rearward driving force of the central rod assembly 14), under the action of the rearward driving force of the central rod assembly 14, the central rod assembly 14, the pull rod assembly 13, and the first magnet 11 move rearward until returning to the initial state. In this case, the jaw portion 200 and the shaft portion 100 are separated.

The magnetically controlled forceps provided in this solution can be operated entirely with one hand.

### Embodiment 2

A pushing device for a magnetically controlled forceps provided in this embodiment has a structure roughly the same as the structure provided in Embodiment 1, except that in this embodiment, a central rod assembly 14 and a pull rod assembly 13 are completely separate structures, namely, no push-pull linkage is formed. In addition, driving ends of the central rod assembly 14 and the pull rod assembly 13 are also separate. In this specific embodiment, two handles (not shown) are used to link with the central rod assembly 14 and the pull rod assembly 13, respectively, namely, one handle is linked with the central rod assembly 14, and the other handle is linked with the pull rod assembly 13. Different handles are controlled, so that actions of a first magnet 11 and a sliding member 12 are controlled, respectively. Of course, the handles may alternatively be replaced by other driving components.

### Embodiment 3

A pushing device for a magnetically controlled forceps provided in this embodiment has a structure roughly the same as the structure provided in Embodiment 1, except that in this embodiment, an actuation module includes only a central rod assembly 14. A connection between the central rod assembly 14 and a sliding member 12 is the same as a connection in Embodiment 1. In addition, the central rod assembly 14 is also linked with a first magnet 11 in the following manner: the first magnet 11 is at a foremost position in an initial state; and the central rod assembly 14 does not push the first magnet 11 when moving forward; or the central rod assembly 14 can drive, when moving backward, the first magnet 11 to move rearward. It can be understood as follows: although the central rod assembly 14 does not push the first magnet 11 when moving forward, the central rod assembly 14 can form a limit with the first magnet 11; when moving rearward, the central rod assembly 14 synchronously drives the first magnet 11 rearward through the limit, and then releases the limit, so that the first magnet 11 returns to the foremost position in the initial state.

The above can be implemented in various ways, such as providing a controllable snap limit structure on the central rod assembly 14. When the central rod assembly 14 moves forward, the snap limit structure is controlled to snap onto the first magnet 11, thus limiting the first magnet 11. After the first magnet 11 is pulled rearward for a specific distance, the snap limit structure is released by control, allowing the first magnet 11 to reset.

In the magnetically controlled forceps provided in this embodiment, the above pushing device for the magnetically controlled forceps is used as a shaft portion 100. When the shaft portion 100 approaches a jaw portion 200, the first magnet 11 and the second magnet 24 are directly magnetically attracted and fixed. When separation is required, the central rod assembly 14 is pulled rearward, thereby driving the first magnet 11 rearward and away from the second magnet 24, so that the shaft portion 100 and the jaw portion 200 are separated.

Although the present invention is specifically demonstrated and introduced with reference to preferred implementations, it should be understood by those skilled in the art that all changes made to the present invention in form and detail within the spirit and scope of the claims of the present invention as defined by the attached claims shall fall within the protection scope of the present invention.

## Claims

1. A pushing device for a magnetically controlled forceps, comprising an actuation module, a sliding member, and a first magnet, wherein the sliding member is located around a periphery of the first magnet, and the actuation module is in driving connection with the sliding member to drive the sliding member to move back and forth.

2. The pushing device for the magnetically controlled forceps according to claim 1, **characterized in that** the actuation module comprises a central rod assembly and a pull rod assembly; the first magnet is assembled at a front end of the pull rod assembly to move back and forth when driven by the pull rod assembly; and the sliding member is located around the periphery of the first magnet and connected to the central rod assembly to move back and forth when driven by the central rod assembly.

3. The pushing device for the magnetically controlled forceps according to claim 2, **characterized in that** the pushing device for the magnetically controlled forceps further comprises a support tube, and the central rod assembly, the pull rod assembly, and the first magnet are all disposed in the support tube.

4. The pushing device for the magnetically controlled forceps according to claim 3, **characterized in that** the sliding member is a sliding tube, and the sliding tube is slidably sleeved on an outer wall of the support tube.

5. The pushing device for the magnetically controlled forceps according to claim 2, **characterized in that** the central rod assembly and the pull rod assembly are configured in a push-pull linkage; the pull rod assembly has a forward driving force, the central rod assembly has a rearward driving force, the driving force of the central rod assembly is greater than the driving force of the pull rod assembly and less than a sum of the driving force of the pull rod assembly and a magnetic engagement force of the first magnet when fully magnetically engaged.

6. The pushing device for the magnetically controlled forceps according to claim 5, **characterized in that** the pull rod assembly comprises a pull rod and a first compression spring, the pull rod is disposed in the first magnet and abuts against a front end of the first magnet, and the first compression spring applies a forward elastic force to the first magnet.

7. The pushing device for the magnetically controlled forceps according to claim 5, **characterized in that** the central rod assembly comprises a central rod, a central rod limit buckle, and a second compression spring, the central rod is fixedly connected to the central rod limit buckle, the central rod is connected to the sliding member, and the second compression spring applies a rearward elastic force to the central rod limit buckle and the central rod.

8. The pushing device for the magnetically controlled forceps according to claim 5, **characterized in that** a limit notch extending in a front-rear direction is formed on the central rod assembly, and the pull rod assembly is provided with a pin and is disposed in the limit notch through the pin, thereby implementing a push-pull linkage between the central rod assembly and the pull rod assembly.

9. The pushing device for the magnetically controlled forceps according to any one of claims 1, 2, or 5, **characterized in that** the pushing device for the magnetically controlled forceps further comprises a movable handle, and the movable handle acts on the actuation module to actuate the actuation module.

10. A magnetically controlled forceps, comprising a jaw portion and a shaft portion that are separable, **characterized in that** the jaw portion comprises a fixed jaw, a movable jaw, and a second magnet, the movable jaw is hinged to the fixed jaw and is provided with a driving arm; the second magnet is connected to the fixed jaw; the shaft portion is the pushing device for the magnetically controlled forceps according to any one of claims 1 to 9; the first magnet is configured to magnetically engage and fix with the second magnet, to fix the jaw portion and the shaft portion; and the actuation module drives a sliding member to pass over a periphery of the second magnet and abut against the driving arm of the movable jaw to actuate the movable jaw.

11. The magnetically controlled forceps according to claim 10, **characterized in that** the second magnet is a solid permanent magnet, and the second magnet is embedded in a rear end of the fixed jaw; and the first magnet is a permanent magnet, and the first magnet and the second magnet are engaged and fixed by opposite poles.
